Europäisches Patentamt

(19) European Patent Office

Office européen des brevets

(11) Publication number: **0 008 214**
**B1**

(12) **EUROPEAN PATENT SPECIFICATION**

(45) Date of publication of patent specification: **02.06.82**

(21) Application number: **79301582.7**

(22) Date of filing: **06.08.79**

(51) Int. Cl.³: **C 12 P 33/16,** C 07 J 1/00
//C12R1/33

(54) Microorganisms and their use in producing androst-4-ene-3,17-dione.

(30) Priority: **07.08.78 US 931741**

(43) Date of publication of application:
**20.02.80 Bulletin 80/4**

(45) Publication of the grant of the patent:
**02.06.82 Bulletin 82/22**

(84) Designated Contracting States:
**CH DE FR GB IT NL**

(56) References cited:
**DE - A - 2 746 383**
**FR - A - 2 345 464**
**US - 3 759 791**

(73) Proprietor: **THE UPJOHN COMPANY**
**301 Henrietta Street**
**Kalamazoo, Michigan 49001 (US)**

(72) Inventor: **Wovcha, Merle Gene**
**145 South Prairie Avenue**
**Kalamazoo, Michigan (US)**
Inventor: **Brooks, Kevin Eugene**
**1135 Greenwood Avenue**
**Kalamazoo, Michigan (US)**

(74) Representative: **Perry, Robert Edward et al,**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Microorganisms and their use in producing androst-4-ene-3,17-dione

This invention relates to two novel microorganisms, one of which can be used to produce the steroid androst-4-ene-3,17-dione (AD) from a 17-($C_{2-10}$ alkyl)steroid.

The transformation of steroids by microorganisms has been widely studied and documented. Apparently, the earliest such work was by Mamoli and Vercellone in 1937, Ber. *70*, 470 and Ber. *70*, 2079. They disclosed the reduction of 17-ketosteroids to 17$\beta$-hydroxy-steroids by fermenting yeast. U.S. Patent Specification No. 2,602,769 discloses the 11$\alpha$-hydroxylation of progesterone with the fungus *Rhizopus nigricans*. U.S. Patent Specification No. 3,684,657 discloses the selective microbiological degradation of 17-alkylsteroids having at least 8 carbons in the 17-alkyl side chain with *Mycobacterium* sp. NRRL B-3683 to prepare AD, androst-1,4-diene-3,17-dione (ADD) and 20$\alpha$-hydroxymethylpregna-1,4-diene-3-one. U.S. Patent Specification No. 3,759,791 discloses the selective microbiological preparation of AD by fermenting a steroid of the cholestane or stigmastane series containing at least 8 carbons in the 17-alkyl side chain with *Mycobacterium* sp. NRRL B-3805 (which has been characterised as *Mycobacterium vaccae*).

German Patent Application P2746383.8 discloses a process for preparing predominantly AD using the microorganism *M. fortuitum* NRRL B-11045. Small amounts of ADD are also produced in the process. We believe this to be the best prior art process for preparing AD.

We have now prepared an adaptive mutant of *Mycobacterium fortuitum* NRRL B-11045 and a novel mutant of the adaptive mutant. The two novel mutants are both of the genus *Mycobacterium fortuitum* and have the accession numbers CBS 313.79 and CBS 314.79. The process of this invention comprises cultivating *M. fortuitum* CBS 313.79 in an aqueous nutrient medium under aerobic conditions in the presence of one or more steroids having a 17-hydrocarbyl side chain containing from 2 to 10 carbon atoms.

*M. fortuitum* CBS 313.79 is characterised by its ability to selectively transform steroids having 17-hydrocarbyl side chains of from 2 to 10 carbon atoms, and accumulate AD as essentially the sole transformed product in the fermentation beer. The best prior art process for preparing AD known to us accumulates about four times more ADD in the fermentation beer than the subject process. A smaller amount of ADD in the fermentation beer facilitates the isolation of the desired product AD.

The mutant can be obtained by using the mutation procedures disclosed herein, although other mutation procedures may be successful. The adaptive mutant of the present invention may be obtained by growing *M. fortuitum* NNRI B-11045 on a suitable medium containing 9$\alpha$-OH AD. A rapid growth colony, identified as an adaptive mutant, is selected. This adaptive mutant, *M. fortuitum* CBS 314.79, is then subjected to nitrosoguanidine (NTG) mutagenesis. The mutant is then selected on agar plates using a 9$\alpha$-OH AD medium, as above. The desired mutant *M. fortuitum* CBS 313.79 will not grow on a medium containing AD or 9$\alpha$-OH AD as the sole carbon source.

The steroid substrates which can be converted to AD by the process of this invention preferably have a 17-alkyl or 17-alkenyl side chain of from 2 to 10 carbon atoms. Examples of suitable steroid substrates are sisterols, cholesterol, stigmasterol and campesterol. The steroid substrates can be in either pure or crude form.

The novel mutants of this invention were deposited with the Centraalbureau voor Schimmelcultures, Baarn, Holland on 18th June, 1979 and given the Accession Nos. 313.79 and 314.79.

M. fortuitum NRRL B-11045 has been deposited in the permanent collection of the Northern Regional Research Laboratory, U.S. Department of Agriculture, Peoria, Illinois, U.S.A.

*M. fortuitum* CBS 313.79 is distinguishable from *M. fortuitum* NRRL B-11045 by its production of AD with lesser amounts of ADD in the fermentation beer.

*M. fortuitum* NRRL B-11045 has been distinguished from the *Mycobacterium* species NRRL B-3805 disclosed in U.S. Patent Specification No. 3,759,791. NRRL B-3805 has the general characteristics of Mycobacterium vaccae which is an example of a species distinctly different from *M. fortuita*. See Bergey's Manual of Determinative Bacteriology, 8th Edition, The Williams and Wilkins Company (1974) 695 and 696 for a comparison of these microorganisms.

The morphology and drug sensitivities of *M. fortuita* CBS 313.79 and 314.79 are indistinguishable from those of *M. fortuitum* ATCC 6842 and *M. fortuitum* NRRL B-11045. Each *M fortuitum* is an acid-fast non-motile, non-spore-forming bacillus belonging to the family *Myco-bacteriaceae* of the order *Actinomycetales*. According to Runyon's classification, Med. Clin. North America *43* (1959) 273, *M. fortuitum* is a non-chromogenic group IV mycobacterium, i.e., it grows rapidly at low temperatures to produce nonpigmented colonies on relatively simple media. The morphologies of *M. fortuita* CBS 313.79 and 314.79 are substantially as given in Bergey's Manual of Determinative Bacteriology, 7th edition (1957) 698—9.

The process of the present invention can be effected in a growing culture of *M. fortuitum*

CBS 313.79 either by adding the selected steroid substrate to the culture during the incubation period, or by incorporating it in the nutrient medium prior to inoculation. The steroid can be added alone or in combination with another steroid. The preferred concentration of the steroid in the culture is 0.1 to 100 grams per litre. The culture is grown in a nutrient medium containing a carbon source such as an assimilable carbohydrate, and a nitrogen source such as an assimilable nitrogen compound or proteinaceous material. Preferred carbon sources include glucose, brown sugar, glycerol, starch, cornstarch, lactose, dextrin and molasses. Preferred nitrogen sources include cornsteep liquor, yeast, autolyzed brewer's yeast with milk solids, soybean meal, cottonseed meal, cornmeal, milk solids, pancreatic digest of casein, fish meal, distiller's solids, animal peptone liquors, meat and bone scraps and ammonium salts. Combinations of these preferred carbon and nitrogen sources can be used advantageously. Trace metals, for example, zinc, magnesium, manganese, cobalt or iron, need not be added to the fermentation medium when tap water and unpurified ingredients are used as components of the medium prior to its sterilisation.

The duration of the process may be from 72 hours to 15 days or more. The incubation temperature is usually from 25 to 37°C, with 30°C being preferred for CBS 313.79. The contents are suitably aerated with sterilised air and agitated to facilitate growth of the micro-organism and thus enhance the effectiveness of the process.

Upon completion of the process, as evidenced by thin layer chromatography (tlc) using silica gel plates (E. Merck, Darmstadt) and a solvent system consisting of 2:3 (by volume) ethyl acetate-cyclohexane, the desired steroid is recovered by known means. For example, the fermentation (transformation) reaction mixture, including the fermentation liquor and cells, can be extracted with a water-immiscible organic solvent for steroids. Suitable solvents are dichloromethane (preferred), methylene chloride, chloroform, carbon tetrachloride, ethylene chloride, trichloroethylene, ether, amyl acetate and benzene.

Alternatively, the fermentation liquor and cells may first be separated by conventional methods, e.g., filtration or centrifugation, and then separately extracted with suitable solvents. The cells can be extracted with either water-miscible or water-immiscible solvents. The fermentation liquor, freed of cells, can be extracted with water-immiscible solvents.

The extracts can be filtered through diatomaceous earth and the filtrate vacuum distilled to dryness. The resulting residue containing the desired transformed steroid then can be dissolved in a minimum of ethyl acetate-cyclohexane (20:80 v/v). This solution then can be chromatographed on silica gel. AD can be separated from the silica gel by elution with the solvent system ethyl acetate-chloroform (15:85 v/v). The compound then can be isolated by evaporation of the solvent and recrystallisation from hexane.

The product of the process of the present invention is AD, a known compound useful as an intermediate in the synthesis of useful steroidal hormones. For example, AD can be used to make testosterone according to processes disclosed in U.S. Patent Specifications Nos. 2,143,453; 2,253,798; 2,264,888 and 2,356,154.

The following Examples illustrate the invention. All percentages are by volume unless otherwise noted.

Example 1
M. fortuitum CBS 314.79
M. fortuitum NRRL B-11045 is streaked on an agar plate medium consisting of the following ingredients:

| | |
|---|---|
| $NH_4NO_3$ | 1.0 g |
| $K_2HPO_4$ | 0.25 g |
| $MgSO_4 \cdot 7H_2O$ | 0.25 g |
| NaCl | 0.005 g |
| $FeSO_4 \cdot 7H_2O$ | 0.001 g |
| Distilled Water, q.s. | 1 litre |

The pH is adjusted to 7.0 with 1 $N$ HCl. Agar (15 g/l) and 9$\alpha$-hydroxyandrostenedione (0.25 g/l) are added and the medium is autoclaved for 30 minutes at 121°C. The hot medium is then poured into a sterile blender vessel, blended for several minutes, and then poured into sterile Petri plates. The plates are incubated at 28°C for about 7 days. M. fortuitum CBS 314.79 is selected from the plates. This product demonstrates rapid growth on the plates.

Example 2
M. fortuitum CBS 313.79
(a) Nitrosoguanidine Mutagenesis
A seed medium is prepared from the following:

| | |
|---|---|
| Nutrient Broth | 8 g |
| Yeast Broth | 1 g |
| Glycerol | 5 g |
| Tween 80 | 1 g |
| Distilled Water, q.s. | 1 litre |

(The nutrient broth is that available under the name "Difco". Tween 80 is available from Atlas Refinery Inc., Newark, New Jersey, U.S.A.).

The pH of the medium is adjusted to 7.0 with 1 $N$ NaOH and then sterilised at 121°C for 20 minutes.

Cells of M. fortuitum CBS 314.79 are grown at 28°C on the sterile seed medium to a density of about $5 \times 10^8$ per ml, pelleted by centrifugation, and then washed with an equal volume of sterile 0.1 $M$ sodium citrate, pH 5.6. Washed cells are resuspended in the same volume of citrate buffer, a sample removed for titering (cell count), and nitrosoguanidine added to a final concentration of 50 μg/ml. The cell suspension is incubated at 37°C in a water bath for 30 minutes, after which a sample is again removed for titering and the remainder centrifuged down and washed with an equal volume of sterile 0.1 *M* potassium phosphate, pH 7.0.

A minimal salts medium, minus a carbon source, is prepared from the following:

| | |
|---|---|
| NH₄NO₃ | 1.0 g |
| K₂HPO₄ | 0.25 g |
| MgSO₄ · 7H₂O | 0.25 g |
| NaCl | 0.005 g |
| FeSO₄ · 7H₂O | 0.001 g |
| Distilled Water, q.s. | 1 litre |

The pH of the minimal salts medium is adjusted to 7.0 with 1 *N* HCl and then sterilised at 121°C for 20 minutes. The cells are resuspended in this sterile medium and then plated out to select for mutants.

(b) Selection and isolation

In this step, mutants which are capable of growing on a certain carbon source are distinguished from those which are not capable of growing on the carbon source. In the present case, taking into account the known metabolic pathway from β-sitosterol to AD, the desired mutant is one which will grow on sitosterol but not on test plates containing AD. The test is thus one of growth measurement. Therefore, if the test plate containing AD does not show growth of the mutant, then this is indicative that AD will accumulate in the fermentation beer when using this mutant.

The selection procedure is carried out as follows:

The minimal salts medium described in section (a) but supplemented with glycerol (10 g/l) as the sole source of carbon and energy is prepared. Agar (15 g/l) is added, and the medium is autoclaved at 121°C for 30 minutes and then poured into sterile Petri plates.

Mutagenized cells, as described above, are diluted and spread onto the plates.

Growth on this medium eliminates most nutritional auxotrophs produced by the mutagenesis procedure, e.g. cultures that require vitamins, growth factors, etc. in order to grow on chemically defined medium are eliminated. After incubation at 28°C for about 7 days, the resulting colonies are replicated to test plates suitable for selecting mutants and then back onto control plates containing the glycerol-based medium. The test plates are prepared as described by Peterson, Lewis and Davis (1962) "Preparation of uniform dispersions of cholesterol and other water-insoluble carbon sources in agar media." J. Lipid Research *3*: 275—276. The minimal salts medium in these plates is as described above in section (a) of Example 1.

Agar (15 g/liter) and 9α-OH AD (0.25 g/liter) are added and the resulting suspension autoclaved for 30 minutes at 121°C. The sterile, hot mixture is then poured into a sterile blender vessel, blended for several minutes, and then poured into sterile Petri plates. Foaming tends to be a problem in this procedure but can be reduced by blending when the mixture is hot and by flaming the surface of the molten agar plates. In this manner uniform dispersions of water-insoluble carbon sources are obtained which facilitates the preparation of very homogeneous but opaque agar plates.

Colonies which grew on the control plates, but which showed little if any growth on test plates containing 9α-OH AD as the sole carbon source, are purified by streaking onto nutrient agar plates. After growth at 28°C, individual clones are picked from the nutrient agar plates with sterile toothpicks and retested by inoculating gridded plates containing 9α-OH AD as the carbon source. Purified isolates which still exhibit a phenotype different from the parental culture are then evaluated in shake flasks.

(c) Shake flask evaluation

A seed medium is prepared, adjusted to pH 7.0 and sterilised exactly as in section (a), using 500 ml flasks containing 100 ml of the medium.

The purified isolates from section (b) are grown on agar slates at 28°C. A loop of cells taken from a slant is used to inoculate one of the flasks which is then incubated at 28°C for 72 hours.

Shake flasks (500 ml) are prepared each containing 100 ml of biotransformation medium consisting of the following ingredients:

| | |
|---|---|
| Cerelose | 5.0 g |
| NH₄Cl | 3.0 g |
| KH₂PO₄ | 0.5 g |
| CaCO₃ | 3.0 g |
| Na₃C₆H₅O₇ · 2H₂O | 3.0 g |
| MgSO₄ · 7H₂O | 2.0 g |
| Urea | 0.5 g |
| Tween 80 | 2.0 g |
| Ucon | 8.0 g |
| Tap water, q.s. | 1 litre |

(Ucon is a synthetic antifoam available from Union Carbide Chem. Co. New York, N.Y., U.S.A.).

Soyflour (1 g/l), and then sitosterol (30 g/l), are blended into the medium. The pH of the biotransformation medium is adjusted to 7.0 with 1 *N* NaOH and then the flasks are autoclaved for 30 minutes at 121°C, after which they are cooled to 28°C and are then each inoculated with 10 ml of the seed growth.

The flasks are then incubated at 28 to 30°C on a rotary shaker and samples are taken at intervals. Ten ml samples are removed and extracted by shaking with 3 volumes of methylene chloride. Portions of the extracts are analyzed by tlc using silica gel and the solvent system described above, i.e., 2:3 ethyl acetate-cyclohexane, and by gas-liquid chromatography. Evidence of the presence of AD confirms the selective degradation of sisterol by the mutant produced from *M. fortuitum* CBS 314 . 79.

Example 3 AD

The transformation medium used is the same as in Example 2(c). This medium is sterilised by autoclaving for 30 minutes at 121°C, whereupon it is cooled to 30°C and then inoculated with 10 ml of a seed culture of the mutant *M. fortuitum* CBS 313 . 79 prepared as described in Example 2(c). The inoculated mixture is incubated at 30°C for 336 hours with agitation to promote submerged growth. Following incubation, the mixture is extracted with dichloromethane. The extract is dried over anhydrous sodium sulfate and the solvent is removed by vacuum distillation. The resulting residue is dissolved in a minimum of ethyl acetate-cyclohexane (20:80). This solution is then chromatographed on silica gel. The presence of AD and a very small amount of androst-1,4-diene-3,17-dione is shown by tlc. These compounds are separated from the silica gel by elution with the solvent system ethyl acetate-chloroform (15:85). The compounds are then isolated by evaporation of the solvent and recrystallisation from hexane.

Results similar to those of Example 3 can be achieved by replacing sitosterol by, or adding thereto, any of the steroid substrates named above, or a mixture thereof.

It is pointed out, that such denominations as "Tween" and "Ucon" are registered trade marks.

## Claims

1. A novel laboratory mutant which is *Mycobacterium fortuitum* CBS 313 . 79 or *Mycobacterium fortuitum* CBS 314 . 79.

2. *Mycobacterium fortuitum* CBS 314 . 79.

3. *Mycobacterium fortuitum* CBS 313 . 79.

4. The microorganism of claim 2 as obtained by adaptive mutation of *Mycobacterium fortuitum* NRRL B-11045.

5. The microorganism of claim 3 as obtained by mutation of the micro-organism of claim 2.

6. A process for preparing androst-4-ene-3,17-dione which comprises cultivating a microorganism of the genus *Mycobacterium fortuitum* in an aqueous nutrient medium under aerobic conditions in the presence of one or more steroids having a 17-alkyl side chain containing from 2 to 10 carbon atoms, characterised in that the microorganism is CBS 313 . 79.

7. A process according to claim 6 wherein the steroid, or one of the steroids, is sitosterol.

8. A process according to claim 6 wherein the steroid, or one of the steroids, is cholesterol.

9. A process according to claim 6 wherein the steroid, or one of the steroids, is stigmasterol.

10. A process according to claim 6 wherein the steroid, or one of the steroids, is campesterol.

## Patentansprüche

1. Ein neuer Laboratoriumsmutant, welcher Mycobacterium fortuitum CBS 313 . 79 oder Mycobacterium fortuitum CBS 314 . 79 ist.

2. Mycobacterium fortuitum CBS 314 . 79.

3. Mycobacterium fortuitum CBS 313 . 79.

4. Mikroogranismus nach Anspruch 2, erhalten durch adaptive Mutation von Mycobacterium fortuitum NRRL B-11045.

5. Mikroorganismus nach Anspruch 3, erhalten durch Mutation des Mikroorganismus nach Anspruch 2.

6. Verfahren zur Herstellung von Androst-4-en-3,17-dion, wobei ein Mikroorganismus des genuinen Mycobacterium fortuitum in einem wässrigen Nährmedium unter aerobischen Bedingungen in der Gegenwart eines oder mehrerer Steroide mit einer 17-Alkyl-Seitenkette mit 2 bis 10 Kohlenstoffatomen gezüchtet wird, dadurch gekennzeichnet, dass der Mikroorganismus CBS 313 . 79 ist.

7. Verfahren nach Anspruch 6, dadurch gekennzeichnet, dass das Steroid oder eines der Steroide Stoisterol ist.

8. Verfahren nach Anspruch 6, dadurch gekennzeichnet, dass das Steroid oder eines der Steroide Cholesterol ist.

9. Verfahren nach Anspruch 6, dadurch gekennzeichnet, dass das Steroid oder eines der Steroide Stigmasterol ist.

10. Verfahren nach Anspruch 6, dadurch gekennzeichnet, dass das Steroid oder eines der Steroide Campesterol ist.

## Revendications

1. Nouveau mutant de laboratoire, qui est le *Mycobacterium fortuitum* CBS 313 . 79 ou *Mycobacterium fortuitum* CBS 314 . 79.

2. *Mycobacterium fortuitum* CBS 314 . 79.

3. *Mycobacterium fortuitum* CBS 313 . 79.

4. Le micro-organisme suivant la revendication 2, obtenu par mutation adaptative de *Mycobacterium fortuitum* NRRL B-11045.

5. Le micro-organisme suivant la revendication 3, obtenu par mutation du micro-organisme suivant la revendication 2.

6. Procédé de préparation d'androst-4-ène-3,17-dione, qui consiste à cultiver un micro-organisme du genre *Mycobacterium fortuitum* dans un milieu nutritif aqueux dans des conditions aérobies en présence d'un ou plusieurs stéroïdes ayant une chaîne latérale 17-alkyle contenant 2 à 10 atomes de carbone, caractérisé en ce que le micro-organisme est CBS 313 . 79.

7. Procédé suivant la revendication 6, dans lequel le stéroïde, ou l'un des stéroïdes, est le sitostérol.

8. Procédé suivant la revendication 6, dans lequl le stéroïde, ou l'un des stéroïdes, est le cholestérol.

9. Procédé suivant la revendication 6, dans lequel le stéroïde, ou l'un des stéroïdes, est le stigmastérol.

10. Procédé suivant la revendication 6, dans lequel le stéroïde, ou l'un des stéroïdes, est le campestérol.